# EUROPEAN PATENT APPLICATION

(11) **EP 4 394 349 A1**
(43) Date of publication of application: **03.07.2024**
(21) Application number: 22862871.5
(22) Date of filing: 04.07.2022
(51) Int. Cl.: G01N 15/10, C12M 1/00, C12N 5/00

(54) **METHOD AND APPARATUS FOR SORTING AND IDENTIFYING SINGLE CELL**

(30) Priority: 02.09.2021 CN 202111027657
(71) Applicant: Shanghai Aurefluidics Technology Co., Ltd, Shanghai 201800 (CN)
(72) Inventor: WANG, Mengqi, Jiading, Shanghai 201800 (CN); YANG, Miaomiao, Shanghai 201800 (CN); JIANG, Wei, Shanghai 201800 (CN)
(74) Representative: Canzler & Bergmeier Patentanwälte Partnerschaft mbB
(86) International application number: PCT/CN2022/103658
(87) International publication number: WO 2023/029730

(57) **Abstract**

Provided are a method and apparatus for sorting and identifying a single cell (8). The method for sorting and identifying the single cell (8) comprises a liquid supply step, a photographing and identification step of a first nozzle (101), a printing and photographing step of the single cell (8), and a photographing step of a second nozzle (101). According to the method and apparatus for sorting and identifying the single cell (8), on the basis of the microfluidic characteristics of a high-throughput thermal bubble printing chip (1), the single cell (8) is efficiently and softly derived by using an image recognition method, and the single cell (8) having a high single cell rate and high activity can be quickly obtained in large scale. The method for sorting and identifying the single cell (8) identifies the single cell (8) on the basis of cell morphological characteristics, and the obtained single cell (8) has the characteristic of being high in activity; moreover, the method can provide a zeroth-day appearance for the cell, and the zeroth-day appearance is an important criterion for the single cell (8). After completing the sorting of the single cell (8), the apparatus for sorting and identifying the single cell (8) can monitor the growth state of the cell, and screen a target cell according to a fluorescent label, and thus provide a comprehensive solution for cell experiments.

## Description

### FIELD OF DISCLOSURE

The present disclosure relates to the technical field of microfluidics, cell line development, monoclonal antibody screening, and life science instruments, and in particular, to a method and apparatus for sorting and identifying single cells.

### DESCRIPTION OF RELATED ARTS

Cells are the basic units of life activities. Research at the single-cell level can reveal the developmental laws of life activities in depth, and has wide applications in the fields of monoclonal antibody screening, cell line cultivation, and the like. Currently, single-cell separation methods mainly include micropipette aspiration, limited dilution method, microwell array, and microfluidic-based sorting methods. The limited dilution method requires manual optimization of the dilution ratio, resulting in high workload and poor stability. The cost of the flow cytometry instrument is high, and its use of electrical signals to sort cells will cause great damage to cells. The microwell array method relies on the Poisson distribution, resulting in very low efficiency. Other microfluidic-based sorting methods also have problems such as low throughput, high cost of consumables, low single-cell capture rate, and low single-cell activity.

### SUMMARY OF THE PRESENT INVENTION

The present disclosure provides a method and apparatus for sorting and identifying single cells to solve the problems of low throughput, high cost of consumables, low single-cell capture rate, and low single-cell viability in existing single-cell sorting methods.

The method for sorting and identifying single cells, includes the following steps:
a liquid supplying step: adding a solution containing cells to multiple nozzles of a thermal bubble printing chip;
a first nozzle imaging and recognizing step: capturing an image of the thermal bubble printing chip from a nozzle side of the thermal bubble printing chip by using an optical module to obtain a before-single-cell-printing nozzle image, and obtaining serial numbers of nozzles having single cells based on the before-single-cell-printing nozzle image;
a single cell printing and imaging step: sequentially expelling the single cells from the corresponding nozzles in a predetermined order based on the serial numbers, to a cell receiving device by using a printing method, and capturing a day-zero image of each printed single cell in the cell receiving device by using the optical module; and
a second nozzle imaging step: capturing an image of the thermal bubble printing chip from the nozzle side of the thermal bubble printing chip by using the optical module, to obtain an after-single-cell-printing nozzle image.

Optionally, the liquid supplying step includes ejecting and renewing the solution at a front end of the multiple nozzles of the thermal bubble printing chip.

Optionally, the first nozzle imaging and recognizing step includes:
a nozzle recognition step: searching for the multiple nozzles of the chip according to nozzle image characteristics, and sequentially selecting each of the multiple nozzles; and
a single cell recognition step: performing at least one single cell recognition on the selected nozzle.

Optionally, the single cell recognition step includes:
a first screening step: performing a first recognition of cells in the selected nozzle by adopting a preset image algorithm, if the selected nozzle is preliminarily determined to be qualified in terms of cells it contains, a second screening step is performed on the selected nozzle, or if not qualified, the selected nozzle is excluded for the second screening step;
a second screening step: using a pre-trained neural network to perform a second recognition of cells in preliminarily qualified nozzles so as to further exclude nozzles that have contaminants, cell fragments, or non-cells, where if it is determined that a preliminarily qualified nozzle does contain a single cell, the corresponding serial number is calculated, or if it is determined that this preliminarily qualified nozzle does not actually contain a single cell, this nozzle is excluded.

Optionally, in the first nozzle imaging and recognizing step, if the recognition result shows that there is no single cell in any one of the multiple nozzles of the printing chip, returning to the liquid supplying step.

Optionally, in the single cell printing and imaging step, before expelling each to-be-printed single cell to the cell receiving device, focusing the optical module on the expected landing point of the to-be-printed single cell.

Optionally, in the single cell printing and imaging step, after the single cell in one nozzle is printed, adding culture medium to a well receiving that single cell of the cell receiving device to enhance the activity of the printed single cell.

Optionally, after the second nozzle imaging step, determining whether the number of single cells in the cell receiving device meets a preset requirement, if so, ending single cell sorting, or if not, returning to the liquid supplying step.

Optionally, the method for sorting and identifying single cells further includes a nozzle infiltrating step before the liquid supply step.

Optionally, the cell receiving device includes a well plate.

Optionally, the method for sorting and identifying single cells further includes a cell state monitoring step, where the cell state monitoring step includes: after printing a predetermined number of single cells in the cell receiving device, scanning and imaging the cell receiving device by using the optical module to monitor the cell state.

Optionally, the method for sorting and identifying single cells further includes a secretion detecting step, where the secretion detecting step includes: after printing a predetermined number of single cells in the cell receiving device, capturing fluorescent images of cell secretions in the cell receiving device by using the optical module, to locate a target cell.

The present disclosure also provides an apparatus for sorting and identifying single cells, including a thermal bubble printing chip, an optical module, and a control component, where the apparatus for sorting and identifying single cells is configured to perform the method for sorting and identifying single cells.

As described above, the method and the apparatus for sorting and identifying single cells of the present disclosure are based on a high-throughput microfluidic design of thermal bubble printing chip and image recognition, which can expel the single cells gently and efficiently, thereby obtaining a high volume of single cells with high single cell yield and high single cell activity quickly. The method recognizes single cells based on cellular morphological features, which contributes to obtaining single cells with high activity. Furthermore, the method can provide the appearance of single cells on day zero, which is an important criterion for single cell analysis. In addition, the apparatus for sorting and identifying single cells of the present disclosure provides a comprehensive solution for cellular experiments by monitoring the cell growth and locating the target cells based on fluorescent markers after completing single cell sorting.

### BRIEF DESCRIPTION OF THE DRAWINGS

FIG. 1 shows a flowchart of a method for sorting and identifying single cells of the present disclosure.
FIG. 2 shows an image recognition logic diagram of the method for sorting and identifying single cells of the present disclosure.
FIG. 3 shows a single cell printing timing flowchart of the method for sorting and identifying single cells of the present disclosure.
FIG. 4 shows a schematic diagram of an automated instrument containing control components in which a thermal bubble printing chip is equipped of the present disclosure.
FIG. 5 shows a schematic diagram of an optical module when capturing images of the thermal bubble printing chip from a nozzle side of the thermal bubble printing chip of the present disclosure.
FIG. 6 shows a schematic diagram of the nozzles before printing a single cell.
FIG. 7 shows a schematic diagram of the thermal bubble printing chip printing a solution containing single cells to a well plate and the optical module focusing below the well plate on expected landing points of the printed single cells.
FIG. 8 shows a schematic diagram of newly printed single cell droplets containing single cells.
FIG. 9 shows a schematic diagram of a monitoring interface used in cell growth detection.
FIG. 10 shows a schematic diagram of cells inside a well on day zero.
FIG. 11 shows a schematic diagram of cells inside the well on day one.
FIG. 12 shows a schematic diagram of cells inside the well on day three.

### Reference Numerals

- 1: Thermal bubble printing chip
- 101: Nozzle
- 2: Automated instrument
- 201: Base
- 202: Control component
- 203: Reservoir
- 3: Optical module
- 4: Cell
- 5: Solution
- 6: Well plate
- 7: Droplet
- 8: Single cell

### DETAILED DESCRIPTION OF THE EMBODIMENTS

The implementation of the present disclosure will be described in detail by specific embodiments, and those skilled in the art can easily understand other advantages and effects of the present disclosure from the content in this specification. The present disclosure can also be implemented or applied through other different specific embodiments. The details in this specification can also be modified or changed based on different perspectives and applications without departing from the spirit of the present disclosure.

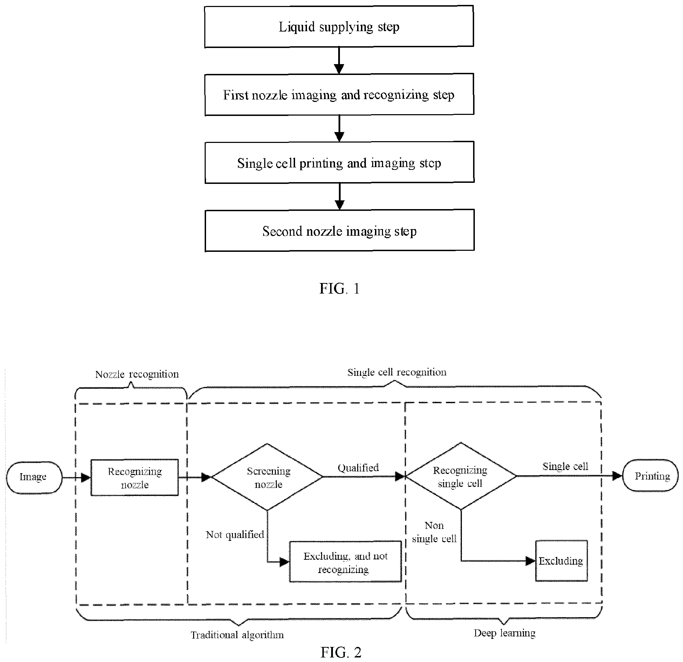

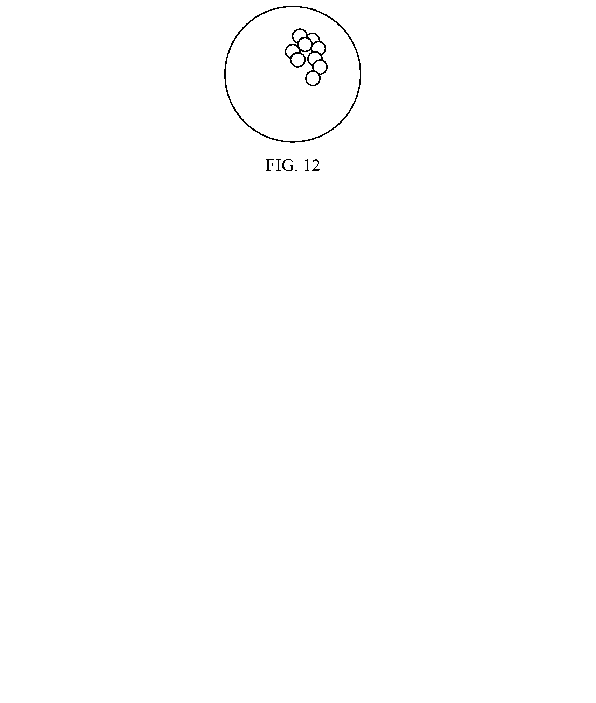

Please refer to FIG. 1 to FIG. 12. It should be noted that the drawings provided in these embodiments are only intended to illustrate the basic concept of the present disclosure. Therefore, only the components related to the present disclosure are shown in the drawings, and are not necessarily drawn according to the actual number, shape, and size of the components in the actual implementation. The types, quantity, and proportions of the components in the actual application may be subjected to arbitrary changes, and the layout of the components may also be more complex.

### Embodiment 1

In this embodiment, a method for sorting and identifying single cells is provided. Please refer to FIG. 1, which shows a flowchart of the method, and the method includes a liquid supplying step, a first nozzle imaging and recognizing step, a single cell printing and imaging step, and a second nozzle imaging step.

Specifically, the liquid supplying step includes adding a solution containing cells to multiple nozzles of a thermal bubble printing chip. The thermal bubble printing nozzles utilize the instant high temperature of a heating film to vaporize liquid above it, generating bubbles to propel the liquid flow out of the nozzle. Subsequently, the liquid is supplemented under the action of capillary force, thereby providing power for the continuous flow of the liquid. The thermal bubble printing nozzles are controlled by the bottom circuit.

As an example, the liquid supplying step includes ejecting and renewing a solution in the nozzles.

As an example, the thermal bubble printing chip is provided with multiple channels communicated to the nozzles, and the solution containing cells can be supplied to the nozzles through the channels. The layout of the channels and the size of the nozzles can be adjusted according to the size of the to-be-sorted cells. An appropriate volume and concentration of cells are added to the thermal bubble printing chip of the present disclosure. By using the high-throughput and single-cell level microfluidic design of the thermal bubble printing chip, a large number of single cells can be formed at once in the nozzles.

As an example, prior to the liquid supplying step, a nozzle infiltrating step is also included in the method for sorting and identifying single cells to reduce negative effects on cell activity. The nozzle infiltrating step may include adding a surfactant to the nozzles to infiltrate the nozzles, and adding a buffer to the nozzles to clean the nozzles by printing, where the buffer may be water or media.

Specifically, the first nozzle imaging and identifying step includes capturing an image of the thermal bubble printing chip from a nozzle side of the thermal bubble printing chip by using an optical module to obtain a before-single-cell-printing nozzle image, and obtaining serial numbers of the nozzles having single cells based on the before-single-cell-printing nozzle image.

As an example, the optical module includes a high-resolution, low-magnification, and long-working-distance object lens, a barrel lens, a coaxial collimation light source, a multi-channel laser light source, a multi-channel filter, a large-target-area camera, etc., and its connection relationship and parameters can be adjusted as needed to achieve large field of view and high-resolution imaging performance.

As an example, obtaining serial numbers of the nozzles having single cells based on the before-single-cell-printing nozzle image includes preferentially selecting the single cells within the image using an image algorithm, and calculating the serial numbers of the nozzles on the printhead to ensure that the single cells are correctly exported from the printhead. The image algorithm herein can combine traditional image recognition and deep learning to provide a more effective solution for different recognition targets.

As an example, FIG. 2 shows an image recognition logic diagram, in which nozzle recognition and single cell recognition are sequentially performed based on the before-single-cell-printing nozzle image. The nozzle recognizing step includes searching for the multiple nozzles of the chip according to nozzle image characteristics, and sequentially selecting each of the multiple nozzles, and the single cell recognizing step includes performing at least one single cell recognition on the selected nozzle.

It should be noted that the printing nozzles are arranged in a special way due to the fact that the printing of single cells requires a special design of the printhead of the hot bubble printing chip, and the serial numbers of the nozzles on the printing head are irregularly distributed. In combination with the vibration caused by the automated motion in actual application, as well as the error of optical imaging and other comprehensive reasons, micron-level errors cannot be avoided, which may lead to the calculation error of the serial numbers of the nozzles containing single cells. In this embodiment, in the nozzle recognizing step, the nozzle searching is first performed according to the nozzle image characteristics, which are repaired through algorithm according to the morphological characteristics of nozzles, and then used for selection of real nozzles. The obtained actual calculated value is used to calculate the serial numbers of the nozzles, which can correctly drive the single cell printing.

As an example, traditional algorithms or other suitable algorithms can be used to recognize nozzles. The traditional algorithms include selection based on morphological features, filtering algorithms, and physical position selection based on the printhead pattern. Physical position selection based on the printhead pattern can exclude non-nozzles, and selection based on morphological features and filtering algorithms can exclude abnormal nozzles and nozzles without cells.

A single cell is a living organism, and there are individual differences in the appearance of living organisms; in addition, cells are transparent, and solutions containing cells are also transparent; therefore, it is very difficult to identify a single cell. In this embodiment, in order to improve the identifying rate, traditional algorithms can be combined with deep learning in the single cell recognizing step.

As an example, the single cell recognizing step includes a first screening step and a second screening step. The first screening step includes performing a first recognition of the cells in the selected nozzle by adopting a preset image algorithm. If the selected nozzle is preliminarily determined to be qualified in terms of cells it contains, the second screening step is performed on the selected nozzle, or if not qualified, the selected nozzle is excluded for the second screening step. In this embodiment, the first screening step preferably uses the traditional algorithms to quickly and roughly determine whether there is a single cell in each selected nozzle. The second screening step uses a pre-trained neural network to perform the second recognition of cells in the preliminarily qualified nozzles so as to further exclude nozzles with contaminants, cell fragments, or non-cells. If it is determined that there is a preliminarily qualified nozzle does contain a single cell, the corresponding serial number is calculated. If it is determined that this preliminarily qualified nozzle does not actually contain a single cell, this nozzle is excluded. In this embodiment, the algorithm in the second screening step can be used to select the single cells of appropriate size and optimal activity state. With this screening mechanism, the single cell yield can reach 95%, and the viability of the selected single cells can reach 90%.

As an example, in the first nozzle imaging and recognizing step, if the recognition result shows that there is no single cell in any of the multiple nozzles of the printing chip, returning to the liquid supplying step.

Specifically, the single cell printing and imaging step includes sequentially expelling the single cell from the corresponding nozzle in a predetermined order based on the numbering of these nozzles, to a cell receiving device by using a printing method, and capturing a day-zero image of each printed single cell in the cell receiving device by using the optical module.

As an example, in the single cell printing and imaging step, before expelling each to-be-printed single cell to the cell receiving device, focusing the optical module on the expected landing point of the to-be-printed single cell, so that the newly printed single cell can be accurately photographed, generating the day-zero image of that cell. The day-zero image of the cell is an important criterion for determining single cells, can verify the results of cell printing when needed, and is crucial for the development of single-cell-related research.

As an example, the cell receiving device includes a well plate with multiple wells arranged according to a predetermined rule, and each well is configured to receive a single cell. The bottom of the well is transparent, and the lens of the optical module is focused on the expected landing point of the to-be-printed single cell through the bottom of the well.

As an example, in the single cell printing and imaging step, after the single cell in one nozzle is printed, adding culture medium to the well receiving that single cell of the cell receiving device to enhance the activity of the printed single cell.

Specifically, the second nozzle imaging step includes capturing an image of the thermal bubble printing chip from a nozzle side of the thermal bubble printing chip by using the optical module, to obtain an after-single-cell-printing nozzle image. It should be noted that the second nozzle imaging step is performed after all the single cells identified from one fluid supply have been printed. By comparing the images obtained in the first nozzle imaging and recognizing step and the second nozzle imaging step, the single cell printing result can be determined.

As an example, after the second nozzle imaging step, determining whether the number of single cells in the cell receiving device meets a preset requirement, if so, ending single cell sorting, or if not, returning to the liquid supplying step.

As an example, the method for sorting and identifying single cell may further include a cell state monitoring step, where the cell state monitoring step includes: after printing a predetermined number of single cells in the cell receiving device, scanning and imaging the cell receiving device by using the optical module to monitor the cell state. For example, the entire well plate can be scanned and photographed at any time to monitor cell growth. When the diameter of each well in the well plate is relatively large and the field of view for photography is relatively small, multiple partial images of one well obtained by scanning and photographing can be combined into a complete image of that well.

As an example, the method for sorting and identifying single cells may further include a secretion detecting step, where the secretion detecting step includes after printing a predetermined number of single cells in the cell receiving device, capturing fluorescent images of cell secretions in the cell receiving device by using the optical module, to locate a target cell.

Specifically, since the activity of cells in a non-cultured environment is directly affected by the exposure time, the identified single cells need to be printed into a specified container within a short time to maintain cell activity, and the optimal timing of all steps needs to be assured. In this embodiment, an optimal automated control strategy is used to efficiently expel the single cells from the thermal bubble printing head. Using this optimal control strategy, the time required to print all the single cells in one 96-well plate is less than 5 minutes.

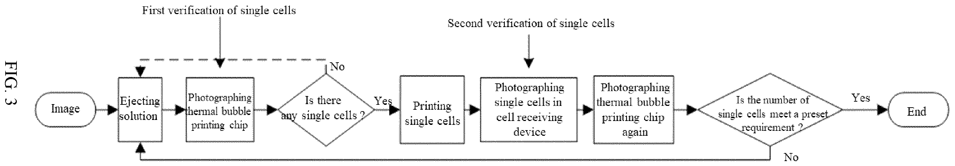

As an example, FIG. 3 shows a single cell printing timing flowchart, in which the following are performed sequentially: ejecting the solutions in the nozzles of the thermal bubble printing head; photographing the thermal bubble printing chip for the first time to determine whether there are single cells, if there is none, returning to the ejecting, or if there is, printing the single cells and photographing the printed single cells for subsequent verification when needed in the future; then photographing the thermal bubble printing chip for the second time; and determining whether the number of printed single cells satisfies the preset requirement, if not, returning to the ejecting, or if so, ending the single cell sorting.

Specifically, before the single cell recognition, solutions in the nozzles of the thermal bubble printing chip are ejected in the waste collection area to update the cell solutions at the front ends of the nozzles. When photographing the thermal bubble printing chip, a high-resolution and wide-field optical module is used, to increase the identifying probability of single cells. Image algorithms are used for calculation, then the results are obtained, and the images are stored. When photographing newly printed single cells, the above-mentioned optical module is focused on the bottom of the well plate to capture images of cells falling to the bottom of the well plate, and image algorithms are used to identify and confirm the single cell printing result. Then, a liquid dispensing probe is used to add an appropriate amount of medium to each well with a single cell in the well plate to ensure high cell viability.

The method for sorting and identifying single cells in this embodiment uses a high-throughput microfluidic design of the thermal bubble printing chip, which can generate multiple single cells at once and can rapidly and gently export the single cells. By multiple times of optical imaging, and large field of view and high-resolution imaging achieved by a low magnification, high resolution, and long working distance optical module, efficient single cell imaging and imaging of single cell printing results are realized. The image algorithms combining multiple methods are used to selectively identify the single cells in the thermal bubble printing head and confirm the single cell printing results, completing double identification of the expelled single cells and achieving a very high single cell yield. With the optimal automated control strategy, the single cells inside the thermal bubble printing head are efficiently expelled, ensuring high cell viability.

### Embodiment 2

This embodiment provides an apparatus for sorting and identifying single cells, including a thermal bubble printing chip, an optical module, and a control component. The apparatus for sorting and identifying single cells can be used to perform the method for sorting and identifying single cells described in Embodiment 1. The control component is used to perform the image algorithm calculation, feedback results, store images, and other calculation operations.

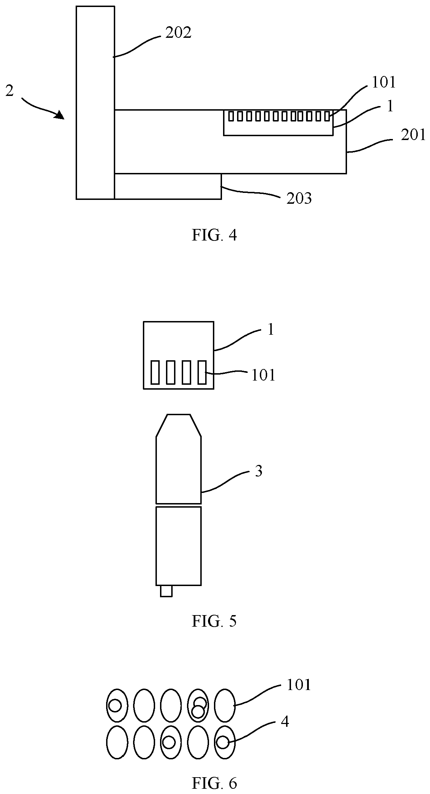

As an example, FIG. 4 shows a schematic diagram of an automated instrument 2 containing the control component in which a thermal bubble printing chip 1 is equipped. The automated instrument 2 includes a base 201, a control component 202, and a reservoir 203 for storing cell solution. The thermal bubble printing chip 1 is arranged on the base 201 and electrically connected to the control component 202. An appropriate volume and concentration of cells are added to the thermal bubble printing chip from the reservoir 203. The high-throughput and single cell level microfluidic design of the thermal bubble printing chip can generate a large number of single cells at once.

As an example, FIG. 5 shows a schematic diagram of an optical module 3 capturing images of the thermal bubble printing chip 1 from a nozzle side of the thermal bubble printing chip 1.

As an example, FIG. 6 shows a schematic diagram of the nozzles before printing single cells, showing the distribution of cells 4 in multiple nozzles 101.

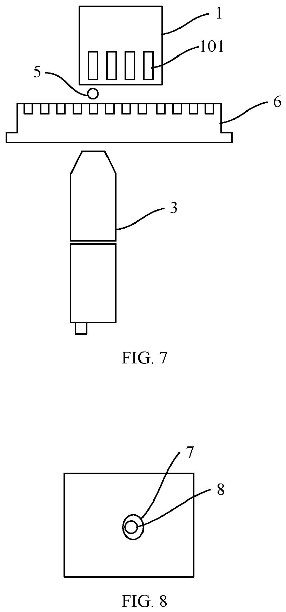

As an example, FIG. 7 shows a schematic diagram of the thermal bubble printing chip 1 printing a solution 5 containing single cells to a well plate 6 and the optical module 3 below the well plate 6 focusing on expected landing points of the printed single cells.

As an example, FIG. 8 shows a schematic diagram of a newly printed single cell droplet 7 containing a single cell 8.

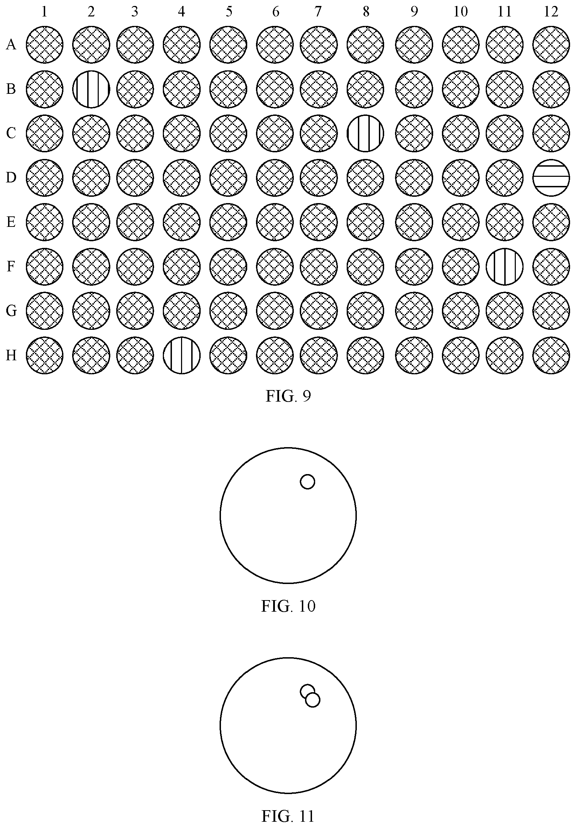

As an example, FIG. 9 shows a schematic diagram of a monitoring interface used in cell growth detection. Each circle represents a well in the well plate, and its position can be determined by row number (e.g., A, B, C, D, E, F, G, H as shown in FIG. 9) and column number (e.g., 1, 2, 3, 4, 5, 6, 7, 8, 9, 10, 11, 12 as shown in FIG. 9). The circles filled with different colors (different patterns in FIG. 9 are used to represent different colors) represent different cell conditions in the wells, such as gray (horizontal stripes) represents no cell in the well, orange (vertical stripes) represents abnormal cell, and green (grid pattern) represents normal cell. Corresponding images of the cell in each well can be obtained after determining the well number.

As an example, after selecting one of the wells in the interface shown in FIG. 9, FIGs. 10, 11, and 12 respectively show the schematic diagrams of the cell in that well on day zero, day one, and day three.

Due to the large field of view and high-resolution imaging performance of the optical module and high-throughput nozzles of the apparatus for sorting and identifying single cells in this embodiment, highly efficient and highly active single cell printing is realized, and multiple identifications can be performed on the expelled cells to obtain a high single cell yield. The optical module engages multiple tasks such as single cell recognition, single cell result identification, and cell growth detection, and can monitor the process of single cell sorting, growth, and characteristic screening.

### Embodiment 3

In this embodiment, the apparatus for sorting and identifying single cells in Embodiment 2 is used for single cell sorting of Chinese hamster ovary (CHO) cells. First, the thermal bubble printing head is installed on the automated instrument containing the control component, and pre-treatment operation is performed on the nozzles. The prepared cell suspension is added to the thermal bubble printing chip, and the automated control strategy is used to perform photographing by the optical module, single cell recognition, and single cell printing, until the single cell printing for the entire 96-well plate is completed. Single cell printing for the 96-well plate takes less than 5 minutes, with a single cell yield of over 90% and post-culture cell viability of up to 80%. When it is necessary to detect cell characteristics, such as cell viability, protein expression, etc., relevant reagents can be added, and target detection can be performed using fluorescence imaging.

In summary, the method and apparatus for sorting and identifying single cells of the present disclosure adopts a high-throughput microfluidic design of thermal bubble printing chip and image recognition, which can expel single cells gently and efficiently, thereby obtaining a high volume of single cells with high single cell yield and high single cell activity quickly. The method recognizes single cells based on cellular morphological features, which contributes to obtaining single cells with high activity. Furthermore, the method can provide the appearance of single cells on day zero, which is an important criterion for single cell analysis. In addition, the apparatus for sorting and identifying single cells of the present disclosure provides a comprehensive solution for cellular experiments by monitoring the cell growth and screening the target cells based on fluorescent markers after completing single cell sorting. Therefore, the present disclosure effectively overcomes various drawbacks of existing technologies and has high industrial utility value.

The above embodiments are merely illustrative of the principles and effects of the present disclosure, and are not intended to limit the scope of the present disclosure. Anyone skilled in the art can modify or change the above embodiments without departing from the spirit and scope of the present disclosure. Therefore, all equivalent modifications or changes made by those skilled in the art in the field of the present disclosure, while not departing from the spirit and technical ideas disclosed in the present disclosure, should still be covered by the claims of the present disclosure.

## Claims

1. A method for sorting and identifying single cells, comprising:
a liquid supplying step: adding a solution containing cells to a plurality of nozzles of a thermal bubble printing chip;
a first nozzle imaging and recognizing step: capturing an image of the thermal bubble printing chip from a nozzle side of the thermal bubble printing chip by using an optical module to obtain a before-single-cell-printing nozzle image, and obtaining serial numbers of the nozzles having single cells based on the before-single-cell-printing nozzle image;
a single cell printing and imaging step: sequentially expelling the single cell from the corresponding nozzle in a predetermined order based on the serial numbers, to a cell receiving device by using a printing method, and capturing a day-zero image of each printed single cell in the cell receiving device by using the optical module; and
a second nozzle imaging step: capturing an image of the thermal bubble printing chip from the nozzle side of the thermal bubble printing chip by using the optical module, to obtain an after-single-cell-printing nozzle image.

2. The method for sorting and identifying single cells according to claim 1, wherein the liquid supplying step comprises ejecting and renewing the solution at a front end of the plurality of nozzles of the thermal bubble printing chip.

3. The method for sorting and identifying single cells according to claim 1, wherein the first nozzle imaging and recognizing step comprises:
a nozzle recognition step: searching for the plurality of nozzles according to nozzle image characteristics, and sequentially selecting each of the plurality of nozzles; and
a single cell recognition step: performing at least one single cell recognition on the selected nozzle.

4. The method for sorting and identifying single cells according to claim 3, wherein the single cell recognition step comprises:
a first screening step: performing a first recognition of cells in the selected nozzle by adopting a preset image algorithm, if the selected nozzle is preliminarily determined to be qualified in terms of cells it contains, a second screening step is performed on the selected nozzle, or if not qualified, the selected nozzle is excluded for the second screening step; and
a second screening step: using a pre-trained neural network to perform a second recognition of cells in preliminarily qualified nozzles so as to further exclude nozzles with contaminants, cell fragments, or non-cells, wherein if it is determined that one of the preliminarily qualified nozzles contains a single cell, the corresponding serial number is calculated, or if it is determined that this preliminarily qualified nozzle does not contain a single cell, this nozzle is excluded.

5. The method for sorting and identifying single cells according to claim 1, wherein in the first nozzle imaging and recognizing step, if the recognition result shows that there is no single cell in any of the plurality of nozzles, returning to the liquid supplying step.

6. The method for sorting and identifying single cells according to claim 1, wherein in the single cell printing and imaging step, before expelling each to-be-printed single cell to the cell receiving device, focusing the optical module on an expected landing point of the to-be-printed single cell.

7. The method for sorting and identifying single cells according to claim 1, wherein in the single cell printing and imaging step, after the single cell in one nozzle is printed, adding culture medium to a well receiving that single cell of the cell receiving device to enhance the activity of the printed single cell.

8. The method for sorting and identifying single cells according to claim 1, wherein after the second nozzle imaging step, determining whether the number of single cells in the cell receiving device meets a preset requirement, if so, ending single cell sorting, or if not, returning to the liquid supplying step.

9. The method for sorting and identifying single cells according to claim 1, further comprising a nozzle infiltrating step before the liquid supply step.

10. The method for sorting and identifying single cells according to claim 1, wherein the cell receiving device comprises a well plate.

11. The method for sorting and identifying single cells according to claim 1, further comprising a cell state monitoring step, wherein the cell state monitoring step comprises: after printing a predetermined number of single cells in the cell receiving device, scanning and imaging the cell receiving device by using the optical module to monitor the cell state.

12. The method for sorting and identifying single cells according to claim 1, further comprising a secretion detecting step, wherein the secretion detecting step comprises: after printing a predetermined number of single cells in the cell receiving device, capturing fluorescent images of cell secretions in the cell receiving device by using the optical module, to locate a target cell.

13. An apparatus for sorting and identifying single cells, comprising a thermal bubble printing chip, an optical module, and a control component, wherein the apparatus for sorting and identifying single cells is configured to perform the method for sorting and identifying single cells according to any one of claims 1-12.
